# EUROPEAN PATENT APPLICATION

(11) **EP 0 621 050 A2**
(43) Date of publication of application: **26.10.1994**
(21) Application number: 94105370.4
(22) Date of filing: 07.04.1994
(51) Int. Cl.: A61M 16/08

(54) **Spiral tube for devices for the artificial ventilation of patients**

(30) Priority: 19.04.1993 IT MI930305 U
(71) Applicant: Mallinckrodt Medical S.p.A., 41037 Mirandola (Modena) (IT)
(72) Inventor: Gibertoni, Lucio, I-41037 Mirandola (Prov. of Modena) (IT)
(74) Representative: Modiano, Guido, Dr.-Ing.

(57) **Abstract**

Spiral tube (1) for devices for the artificial ventilation of patients, comprising: an inner tube (2) connectable between the humidifying and heating unit and the patient; a coil (11) provided on the outer surface of the inner tube; and an outer covering tube (15); thereby forming an insulating layer (10) about the inner tube (2).

## Description

The present invention relates to a spiral tube for devices for the artificial ventilation of patients.

As it is known, in intensive-care wards, during long operations, when the patient is artificially ventilated, heated active humidifiers are used to humidify and heat the dry low-temperature gases that arrive from the ventilator of the respiration unit.

The gases thus heated and humidified are conveyed to the patient over a portion of spiral plastic tube which is generally 1.5-2 meters long.

Considerable heat dissipation occurs along this tube portion, with the consequent forming of condensate inside the tubes, since the relatively warm gases flowing along the tube are saturated with humidity, and cooling therefore unavoidably causes condensation.

It is therefore necessary to interpose, along the inspiratory supply line, condensate traps that must be periodically emptied, consequently increasing the work burden for the nursing personnel.

Another severe problem that directly affects the patient resides in the fact that said patient is fed with unsaturated gases at temperatures that are not always sufficient to ensure normal mucociliary functions.

The principal aim of the present invention is to eliminate the drawbacks described above by providing a new type of spiral tube for devices for the artificial ventilation of patients that allows to significantly reduce the formation of condensate and to supply the patient with a gas in optimum temperature and humidity conditions.

A particular object of the invention is to provide a spiral tube that, while having considerably improved characteristics, has substantially the same weight and flexibility characteristics as the tubes of the known art.

Another object of the present invention is to provide a spiral tube for devices for the artificial ventilation of patients that, due to its particular constructive characteristics, is capable of giving the greatest assurances of reliability and safety in use.

According to the invention, there is provided a spiral tube for devices for the artificial ventilation of patients, comprising an inner tube connectable between the humidifying and heating unit and the patient, characterized in that it comprises an insulating layer arranged around said inner tube.

Further characteristics and advantages of the spiral tube for devices for the artificial ventilation of patients according to the present invention will become apparent from the following detailed description of an embodiment thereof, illustrated only by way of non-limitative example in the accompanying drawings, wherein:
figure 1 is a schematic exploded view of the spiral tube according to the invention, with its elements arranged in layers;
figure 2 is a partially sectional axial view of the tube.

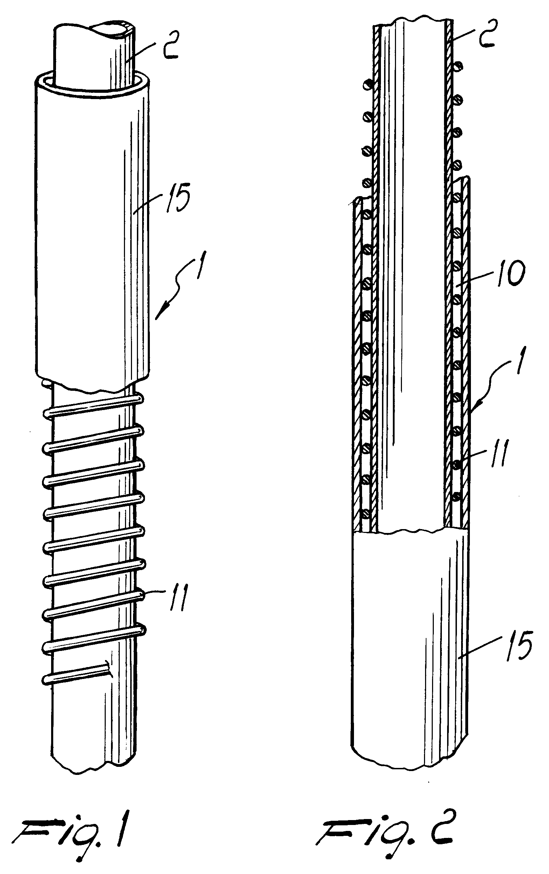

With reference to the above figures, the spiral tube for devices for the artificial ventilation of patients, generally designated by the reference numeral 1, comprises an inner tube 2 that provides the connection for the flow of the humidified and heated gases between the humidifying and heating unit and the patient.

The particularity of the invention resides in the fact that said inner tube 2 externally has an insulating layer constituted by an interspace 10 which is formed by means of a coil 11 arranged outside the inner tube 2 and inside an outer tube 15. In this manner, an air jacket is formed in practice around the inner tube, drastically reducing heat exchange between the ventilation gases and the outside and consequently reducing the condensate inside the inner tube; this condensate, as explained earlier, is normally produced by the sudden decrease in temperature that the saturated and heated gases circulating inside the inner tube undergo.

In this manner one obtains first of all the advantage of reducing the forming of condensate, simplifying all the related operations, and furthermore the patient receives gases that, by virtue of their higher temperature, can have a higher degree of humidity and can consequently be more suitable to ensure normal mucociliary functions.

From what has been described above it can thus be seen that the invention achieves the intended aim and objects, and in particular the fact is stressed that a spiral tube is provided which, by having an interspace, allows to significantly reduce outward heat dissipation, reducing condensation and improving the humidity characteristics of the gas that reaches the patient.

In practice, the materials employed, so long as they are compatible with the specific use, as well as the contingent shapes and dimensions, may be any according to the requirements.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. Spiral tube (1) for devices for the artificial ventilation of patients, comprising an inner tube (2) connectable between the humidifying and heating unit and the patient, characterized in that it comprises an insulating layer (10) arranged around said inner tube.

2. Spiral tube according to claim 1, characterized in that said insulating layer (10) is formed by means of an air jacket.

3. Spiral tube according to the preceding claims, characterized in that said air jacket is formed by a coil (11) which is provided on the outer surface of said inner tube (2) and is arranged inside an outer covering tube (15).
